# EUROPEAN PATENT APPLICATION

(11) **EP 2 738 154 A1**
(43) Date of publication of application: **04.06.2014**
(21) Application number: 12195099.2
(22) Date of filing: 30.11.2012
(51) Int. Cl.: C07C 59/01, C07C 59/125, C07C 59/64, C07D 317/24, C07D 319/06, C10L 1/00, C10L 1/19

(54) **Improved fuel markers**

(71) Applicant: Inter-Euro Technology Limited, Co. Carlow (IE)
(72) Inventor: Parkes, James, Co. Carlow (IE); Parkes, Mary, Co. Carlow (IE)
(74) Representative: Gates, Marie Christina Esther

(57) **Abstract**

There is provided a compound comprising the general structure (I) 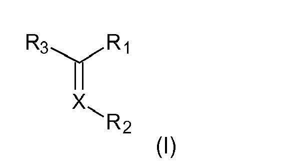 in which, X is N or O;
R³ is OR⁴ or R⁴;
wherein when X is N, R¹ is H, R⁵ or OR⁶; R² is OC(O)R⁷; and R⁴ is R⁵, OR⁵ or aryl, which is unsubstituted or substituted with at least one of R⁵ or OR⁶, OC(O)R⁷, C(O)R⁸, OR⁹, C(H)(OR')₂ or C(R¹⁰)(OR')₂;
wherein when X is O, R² is absent; R¹ is H, R⁵ or OR⁶, CH(OR⁸)R⁹; and R⁴ is R⁵, OR⁵ or aryl, which is unsubstituted or substituted with at least one of R⁵ or OR⁶, OC(O)R⁷, C(O)R⁸, OR⁹, C(H)(OR')₂ or C(R¹⁰)(OR')₂;
wherein R⁵, R⁶, R⁷, R⁸, R⁹, and R¹⁰ are independently selected from linear or branched C₁-C₂₀ alkyl or OH; and
R' is independently selected from linear or branched C₁-C₂₀ alkyl, or may together form a cyclic aliphatic ring having from 2 to 40 carbon atoms.

## Description

### Field of the Invention

The present invention relates to compounds suitable for the use as fuel markers in the marking of lower taxed fuels or other hydrocarbons. Such markers allow revenue authorities to detect use of lower taxed fuels in unapproved situations.

### Background to the Invention

Most countries levy different amounts of taxation on fuels depending on their ultimate usage. For example off road fuels, those used for industrial or agricultural purposes, are often taxed at a relatively lower tax rate and such fuels are often referred to as "rebated fuels". Rebated fuels are generally gas oil or kerosene. On the other hand road fuels are diesel or petrol. Because the off road fuels have lower tax rates they contain visual dyes or other chemical or covert markers. This means that the relevant law enforcement authorities are able to take suspect fuel samples and carry out tests to see if the fuel in question is a rebated fuel or contains a proposition of rebated fuel, which is illegally being used as a road fuel. Typically these roadside spot tests are carried out by HMRC. The samples are treated as forensic samples in the United Kingdom, so they are securely sealed and a chain of evidence provided. Technical reports are issued by the testing laboratory and if court proceedings are issued defence experts have the right to examine the technical data.

The most commonly used markers in rebated kerosene in the United Kingdom are coumarin, Euromarker (which is Cl Solvent Yellow 124) and sulphur. Coumarin is typically added to the fuel at a level of 2 ppm (Kg/million Litres), Euromarker added at a level of 6 to 9 ppm (Kg/million Litres). Sulphur is typically present up to a maximum of 1000 ppm.

In the UK rebated gas oil typically uses as markers Quinizarin at a level of 1.75 ppm (Kg/million Litres), Solvent Red 24 at a level of 4 ppm (Kg/million Litres), Euromarker at a level of 6 to 9 ppm (Kg/million Litres) or Sulphur up to a maximum of 1000 ppm.

Currently analysis of fuels for the coumarin marker involves HPLC or liquid/liquid extraction using sodium hydroxide, or fluorescence spectroscopy to quantify the level of coumarin present, or total luminescence spectroscopy, which also confirms the presence of coumarin. Using these techniques it is possible to confirm the presence of very small amounts of rebated kerosene in a road fuel sample. For rebated gas oil, Quinizarin can be detected by liquid/liquid extraction followed by UV spectroscopy; Solvent Blue 79 can be detected by UV spectroscopy, the Euromarker can be detected by HPLC. However HPLC is increasingly being used to replace other techniques for detection and analysis of all of the above markers, because of its greater sensitivity and the ability to automate the process.

Although not a true "marker", the total sulphur content of a fuel is very a useful indicator of the presence of rebated fuels in any fuel sample. This is because rebated gas oil and kerosene can contain up to a 1000 ppm sulphur whereas diesel and petrol only contain up to 10 ppm sulphur. Sulphur analysis can be carried out by ultra-violet fluorescence (UVF) and is very accurate in the range 10 to 1000 ppm sulphur.

Fuel laundering is the process by which unscrupulous individuals attempt to remove the markers from the rebated fuel so that it can be sold illegally as road fuel.

There are three main types of fuel laundering which take place:

Chemical attack using strong mineral acids such as concentrated Sulphuric Acid or Hydrochloric Acid; Chemical attack using strong alkalis such as caustic soda/sodium hydroxide; Use of absorbents such as Fullers earth, bleaching earth or activated charcoal.

Sometimes a combination of these techniques is used to launder the fuel. In order to combat fuel mis-use, the authorities use highly sensitive analytical methods, which are able to detect very small amounts of markers left after laundering. Typically the samples are profiled using a combination of liquid chromatography and gas chromatography, fluorescence spectroscopy and total sulphur content, which builds up a picture of the suspect sample. It is also known that different markers are affected by fuel laundering to different degrees.

Organic compounds can range from polar to non-polar depending on the bonds within the compound. The greater the electronegativity difference between atoms in a bond the more polar the bond is. In a polar bond a partial negative charge is found on the more electronegative atom and the other atom in the bond has a partial positive charge. The polarity or otherwise of an organic compound has a large impact on its chemical and physical properties. For example, polar organic compounds such as ethanol will be miscible with polar solvents such as water. Non-polar compounds, such as hydrocarbons, for example, hexane or octane or other long chain alkanes of which diesel is composed are not miscible with water or other polar solvents.

Another significant difference between polar and non-polar compounds is their ability to be absorbed onto such relatively polar materials as silica gel or alumina. If a sample of diesel containing markers with a relatively high degree of polarity is passed through these absorbent materials, they will selectively remove the marker from the diesel by the process of absorption.

Markers for marking diesel are based on a number of different classes of organic compounds, azo dyes etc. These are chosen on the basis that they are coloured or can easily be converted to a coloured compound for detection purposes.

More complex structures are also used which give a colour reaction with change in pH or on addition of silica gel as in Crystal Violet Lactone. (McDermott, S.D., Parkes, J., and Kearney, A., "Crystal Violet Lactone as a Security Marker for Fuels" Journal of Forensic Sciences, JFSCA, Vol. 40, No.4, July 199S, pp. 662-663.

Azo solvent dyes are inexpensive and have been used for many years to mark petroleum products. A certain amount of gasoline or diesel has been marked for many years, allowing farmers and industrialists to buy this marked product at a cheaper rate and thus encourage agricultural activity and promote industry. Cars and all other road traffic use the unmarked more expensive fuel.

In UK and formerly in Ireland, Solvent Red 24, has been used along with quinizarin giving "red" diesel as the rebated fuel compared to " white" diesel for road use. This can be extracted using NaOH (IP method).

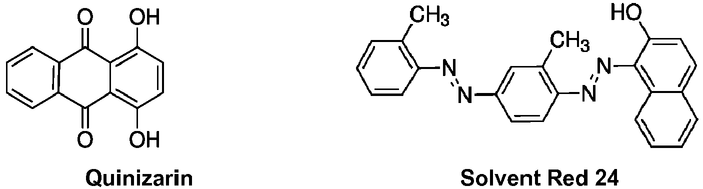

Dyes or markers have been used as a safety measure to show that a particular product is flammable, e.g. violet being added to methylated spirits. Crystal Violet Lactone was used as a security marker in a large company to discover which lorry drivers were using rebated fuel for road use and in their own cars.

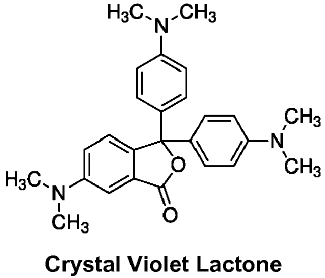

Solvent yellow 124 (Euro Marker) can also be used and which is pale yellow. Colourless markers have also been used which still remain after colour is removed. A roadside test by revenue officials can test diesel in cars by treating a sample with acid to give a water soluble diazo dye which will turn red. If this happens they know that this driver is using rebated fuel in his tank.

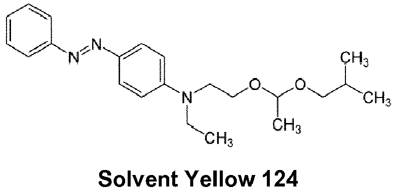

Illegal removal of fuel revenue markers, laundering is a major loss of revenue to Governments.

Laundering methods include treatment with acid, to remove e.g. Euro Marker or to cause breakdown of the complex dye structure or by absorption by passing through high surface area solid absorbents e.g. alumina, silica gel or activated charcoal. This is a form of column chromatography and will separate materials based on presence of polar groups in the marker compared to the less polar diesel or kerosene fuels.

To hinder removal by absorption the marker compounds disclosed in EP Patent 1580 254 A2, by having a long chain alkyl group attached to the benzene ring of the diazo dye to make the compound overall less polar and less prone to removal by absorption.

It is important that a marker for diesel can be initially detected by a roadside test and subsequently analysed in a laboratory to give forensic evidence on which a successful prosecution can be based.
In the past roadside tests were based on extracting a coloured form of the marker into sodium hydroxide, for Quinizarin, or into acid, for Solvent Yellow 124. These detection methods were a weakness in these markers because it showed criminals how the marker could be removed from the diesel during a laundering operation.

### Object of the Invention

The object of the present invention is to provide a marking system for fuels which is resistant to laundering. A further object is to provide a marking system in which the analysis of the marker used in the fuel is easy to automate. A further object is to provide a marker for which a "road side" test is safe, rapid, definitive and cheap to perform. A further object is to provide a marker system for which the analysis can be carried out by a mobile road side unit. A further aspect of the invention is to provide a marker or series of further markers for use in fuels. A further object is to provide suitable marker compounds which can be synthesised from easily available starting materials using basic chemical operations such as esterification, methylation etc.

### Summary of the Invention

The present invention provides organic marker compounds which comprise relatively non-polar functional groups that contain at least one of ether, ester and imodoyl functional groups. Preferred compounds can also contain at least one chiral centre, for example a chiral carbon. The use of relatively low polarity functional groups and optional feature of chirality in the compounds of the invention, means that the compounds of the invention are particularly suitable for use in fuels as markers.

The marker compounds are based on organic compounds in which there is provided at least one of a relatively non polar ether group or ester group, and optionally at least one chiral centre, for example a chiral carbon atom which advantageously enables the compound to be optically active. Preferably, the compounds of the invention do not comprise polar groups such as OH or ionic functionality, for example, halogens.

By relatively non-polar it is meant the following: esters are more polar than ethers but less polar than alcohols. They participate in hydrogen bonds as hydrogen-bond acceptors, but cannot act as hydrogen-bond donors, unlike their parent alcohols. This ability to participate in hydrogen bonding confers some water-solubility. Because of their lack of hydrogen-bond-donating ability, esters do not self-associate. Ether molecules cannot form hydrogen bonds with each other, resulting in a relatively low boiling points compared to those of the analogous alcohols. The difference, however, in the boiling points of the ethers and their isometric alcohols becomes lower as the carbon chains become longer, as the van der Waals interactions of the extended carbon chain dominates over the presence of hydrogen bonding. Ethers are slightly polar. The C-O-C bond angle in the functional group is about 110°, and the C-O dipoles do not cancel out. Ethers are more polar than alkenes but not as polar as alcohols, esters, or amides of comparable structure. However, the presence of two lone pairs of electrons on the oxygen atoms makes hydrogen bonding with water molecules possible.

According to the present invention there is provided a compound comprising the general structure (I) 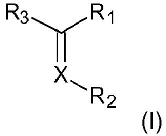
in which, X is N or O;
R³ is OR⁴ or R⁴;
wherein when X is N, R¹ is H, R⁵ or OR⁶; R² is OC(O)R⁷; and R⁴ is R⁵, OR⁵ or aryl, which is unsubstituted or substituted with at least one of R⁵ or OR⁶, OC(O)R⁷, C(O)R⁸, OR⁹, C(H)(OR')₂ or C(R¹⁰)(OR')₂; or
wherein when X is O, R² is absent; R¹ is H, R⁵ or OR⁶, CH(OR⁸)R⁹; and R⁴ is R⁵, OR⁵ or aryl or heteroaryl having at least one N, O or S atom and wherein the aryl or heteroaryl is unsubstituted or substituted with at least one of R⁵ or OR⁶, OC(O)R⁷, C(O)R⁸, OR⁹, C(H)(OR')₂ or C(R¹⁰)(OR')₂;
wherein R⁵, R⁶, R⁷, R³, R⁹, and R¹⁰ are independently selected from linear or branched C₁ - C₂₀ alkyl or OH; and
R' is independently selected from linear or branched C₁ -C₂₀ alkyl, or may together form a cyclic aliphatic ring having from 2 to 40 carbon atoms.
Suitably, R⁵, R⁶, R⁷, R⁸, R⁹, R¹⁰ R¹¹, and R' are independently selected from linear or branched C₁ -C₂₀ alkyl, more preferably, linear or branched C₁ -C₁₅ alkyl, more preferably, linear or branched C₁ -C₁₀ alkyl, more preferably, linear or branched C₁ -C₆ alkyl, more preferably, linear or branched C₁ -C₃ alkyl, more preferably, methyl, ethyl or propyl.

Preferably, the compound comprises the general structure (I) 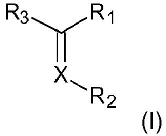
in which, X is N;
R³ is OR⁴ or R⁴;
wherein when X is N, R¹ is R⁵; R² is OC(O)R⁷; and R⁴ is aryl, which is unsubstituted or substituted with at least one of R⁵ or OR⁶, OC(O)R⁷, C(O)R⁸, OR⁹, C(H)(OR')₂ or C(R¹⁰)(OR')₂.=

Preferably, the compound comprises the general structure (I) 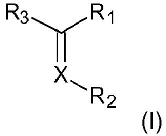
in which, X is O;
R³ is OR⁴ or R⁴;
wherein when X is O, R² is absent; R¹ is H, R⁵ or OR⁶, CH(OR⁸)R⁹; and R⁴ is R⁵, OR⁵ or aryl, which is unsubstituted or substituted with at least one of R⁵ or OR⁶, OC(O)R⁷, C(O)R⁸, OR⁹, C(H)(OR')₂ or C(R¹⁰)(OR')₂.

Preferably, the compound comprises the general structure (I) 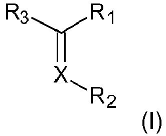
in which, X is N;
R¹ is Methyl, Ethyl, Propyl, Butyl;
R² is OC(O)R⁷;wherein R⁷ is Methyl, Ethyl, Propyl, Butyl;
R³ is aryl, which is unsubstituted or substituted with at least one of R⁵ or OR⁶, OC(O)R⁷, C(O)R⁸, C(H)(OR')₂ or C(R¹⁰)(OR')₂, wherein R⁵, R⁶, R⁷, R⁸, and R¹⁰ are independently selected from linear or branched C₁ -C₂₀ alkyl.

Preferably, the compound comprises the general structure (I) 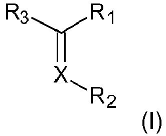
in which, X is O;
R¹ is H, R⁵ or OR⁶, CH(OR⁸)R⁹;
R³ is aryl or Oaryl, which is unsubstituted or substituted with at least one of R⁵ or OR⁶, OC(O)R⁷, C(O)R⁸, OR⁹, C(H)(OR')₂ or C(R¹⁰)(OR')₂, R⁵, R⁶, R⁷, R⁸, and R¹⁰ are independently selected from linear or branched C₁ -C₂₀ alkyl.

Preferably, the compound has general structure (Ia) or (Ib) 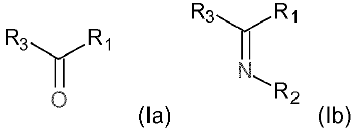 and
wherein R⁵, R⁶, R⁷, R³, R⁹, and R¹⁰ are independently selected from linear or branched C₁ - C₁₀ alkyl; and
R' is independently selected from linear or branched C₁ -C₁₀ alkyl, or may together form a cyclic aliphatic ring having from 2 to 20 carbon atoms.

Preferably, the compound has general structure (Ic) or (Id) or (Ie) or (If) 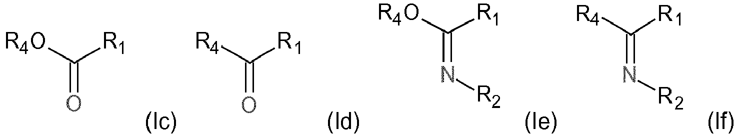 and
wherein R⁵, R⁶, R⁷, R³, R⁹, and R¹⁰ are independently selected from linear or branched C₁ - C₆ alkyl; and
R' is independently selected from linear or branched C₁ -C₆ alkyl, or may together form a cyclic aliphatic ring having from 2 to 12 carbon atoms.

Preferably, the compound has general structure (Ig) or (Ih) or (Ii)

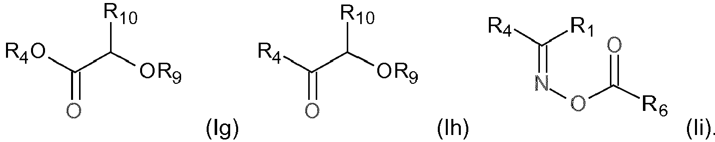

Most preferably, each of R⁵, R⁶, R⁷, R⁸, R⁹, and R¹⁰ are independently selected from a linear or branched C₁ -C₄ alkyl, that is methyl, ethyl, propyl, butyl, or tert-butyl; and R' is independently selected from linear or branched C₁ -C₃ alkyl, or may together form a cyclic aliphatic ring having from 2 to 6 carbon atoms.

The skilled person will appreciate that the alkyl groups may be branched or linear, for example for C1: no isomers: methane, C2: no isomers: ethane, C3: no isomers: propane, C4: 2 isomers: n-butane & isobutene, ·C5: 3 isomers: pentane, isopentane, neopentane, ·C6: 5 isomers: hexane, 2-Methylpentane, 3-Methylpentane, 2,3-Dimethylbutane & 2,2-Dimethylbutane. The skilled person will appreciate that branched alkanes can be chiral. For example 3-methylhexane and its higher homologues are chiral due to their stereogenic center at carbon atom number 3. In addition to these isomers, the chain of carbon atoms may form one or more loops. Such compounds are called cycloalkanes.

Most preferably, R⁵, R⁶, R⁷, R⁸, R⁹, and R¹⁰ are independently CH₃.

Suitably, the aryl group is selected from the group consisting of: phenyl, tolyl, xylyl, naphthyl (which are derived from benzene, toulene, xylene, and naphthalene respectively) and which may be unsubstituted or substituted with at least one of R⁵ or OR⁶, OC(O)R⁷, C(O)R⁸, OR⁹, C(H)(OR')₂ or C(R¹⁰)(OR')₂, where the groups are defined above. Phenyl, tolyl, and xylyl are particularly preferred. Phenyl is the most preferred aryl in the compounds of the invention.

Preferably, some of the ether groups are preferred in the form of an acetal or ketal structure.

In some embodiments, R₄ is preferably aryl, for example phenyl. In such cases the compound may be of general formula type: 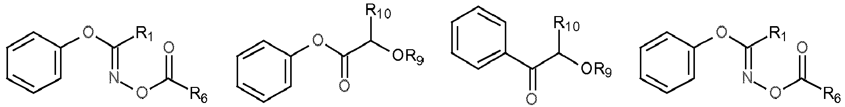 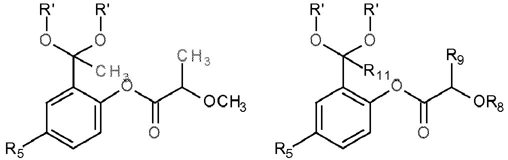 wherein the R¹, R⁶, R⁹ and R¹⁰ are as described above and optionally the phenyl ring is further substituted with at least one of R⁵ or OR⁶, OC(O)R⁷, C(O)R⁸, OR⁹, C(H)(OR')₂ or C(R¹⁰)(OR')₂, wherein R⁵, R⁶, R⁷, R⁸, R⁹, R¹⁰ R¹¹, and R' are independently selected from linear or branched C₁ -C₂₀ alkyl.

In other embodiment, R⁴ is group OR⁵, which is preferably a propylene chain and the compound has general formulas

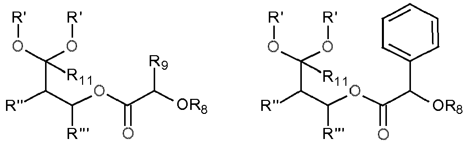

Further compounds include one selected from the group consisting of:

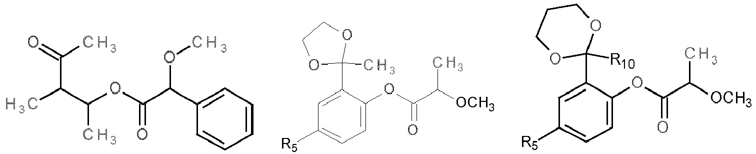

Preferred specific examples include one selected from the group consisting of:

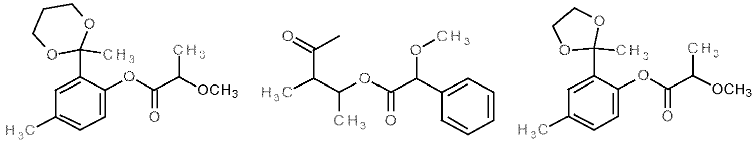

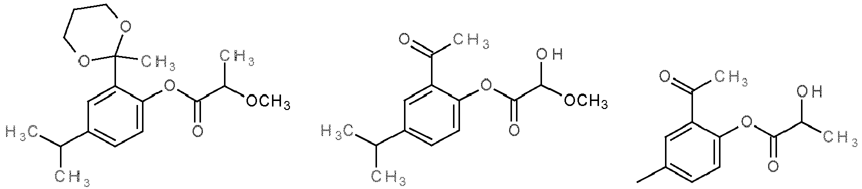

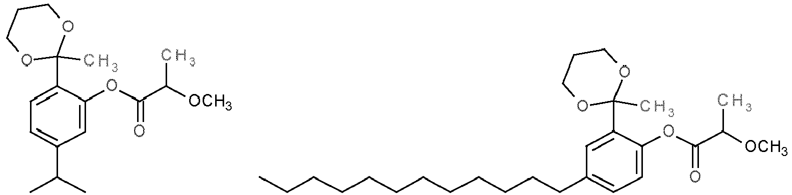

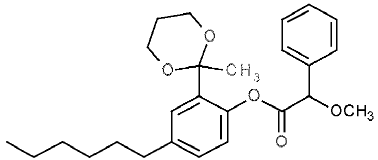

In a related aspect there is provided for use of a compound comprising the general structure (I) 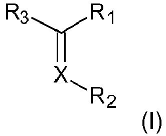
in which, X is N or O;
R³ is OR⁴ or R⁴;
wherein when X is N, R¹ is H, R⁵ or OR⁶; R² is OC(O)R⁷; and R⁴ is R⁵, OR⁵ or aryl, which is unsubstituted or substituted with at least one of R⁵ or OR⁶, OC(O)R⁷, C(O)R⁸, OR⁹, C(H)(OR')₂ or C(R¹⁰)(OR')₂;
wherein when X is O, R² is absent; R¹ is H, R⁵ or OR⁶, CH(OR⁸)R⁹; and R⁴ is R⁵, OR⁵ or aryl, which is unsubstituted or substituted with at least one of R⁵ or OR⁶, OC(O)R⁷, C(O)R⁸, OR⁹, C(H)(OR')₂ or C(R¹⁰)(OR')₂;
wherein R⁵, R⁶, R⁷, R³, R⁹, and R¹⁰ are independently selected from linear or branched C₁ - C₂₀ alkyl; and
R' is independently selected from linear or branched C₁ -C₂₀ alkyl, or may together form a cyclic aliphatic ring having from 2 to 40 carbon atoms,
as a fuel marker.

Suitably, the fuel is diesel, petrol or paraffin fuel.

Preferably, the compound for the use of the invention has general structure (Ia) or (Ib) 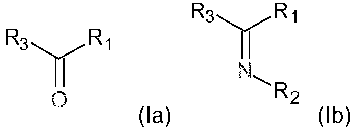 and
wherein R⁵, R⁶, R⁷, R³, R⁹, and R¹⁰ are independently selected from linear or branched C₁ - C₁₀ alkyl; and
R' is independently selected from linear or branched C₁ -C₁₀ alkyl, or may together form a cyclic aliphatic ring having from 2 to 20 carbon atoms.

Preferably, the compound has general structure (Ic) or (Id) or (Ie) or (If) 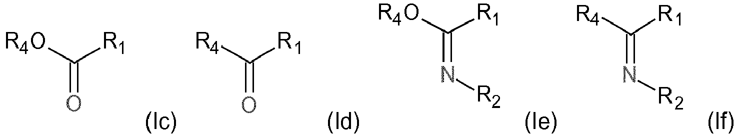 and
wherein R⁵, R⁶, R⁷, R³, R⁹, and R¹⁰ are independently selected from linear or branched C₁ - C₆ alkyl; and
R' is independently selected from linear or branched C₁ -C₆ alkyl, or may together form a cyclic aliphatic ring having from 2 to 12 carbon atoms.

Preferably, the compound for the use of the invention has general structure (Ig) or (Ih) or (Ii) or (Ij)

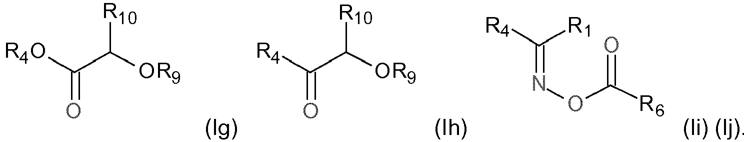

Preferably, in the compound for the use of the invention R⁵, R⁶, R⁷, R⁸, R⁹, and R¹⁰ are independently selected from linear or branched C₁ -C₃ alkyl; and R' is independently selected from linear or branched C₁ -C₃ alkyl, or may together form a cyclic aliphatic ring having from 2 to 6 carbon atoms.

Preferably, in the compound for the use of the invention R4 is preferably aryl, for example phenyl. In such cases the compound may be of general formula type: 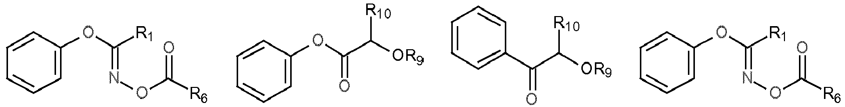 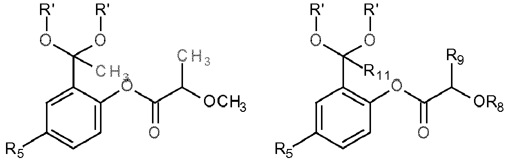 wherein the R¹, R⁶, R⁹ and R¹⁰ are as described above.

In other embodiment, the compound used in the present invention comprises R⁴ being group OR5, which is preferably a propylene chain and the compound has general formulas

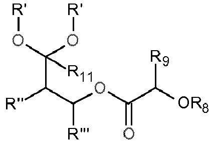

Preferred compounds for use of the invention include one selected from the group consisting of:

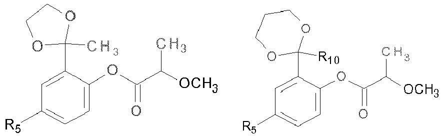

Preferred specific examples include one selected from the group consisting of:

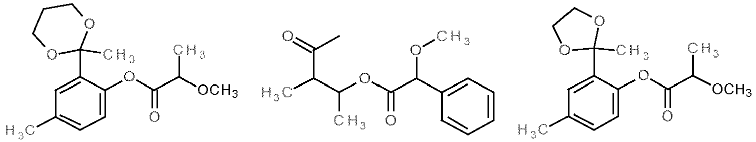

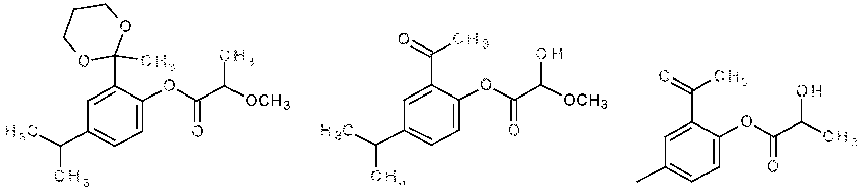

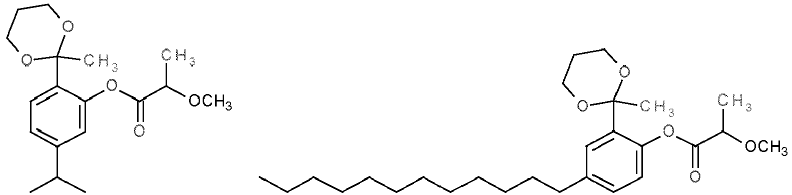

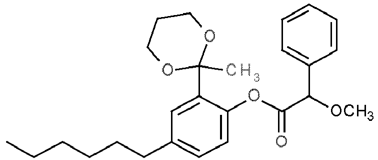

In use, preferably, the compounds of the invention have at least one chiral centre such as a chiral carbon with four different groups attached to it to provide optical activity as a parameter which can be measured and used as an added means of detecting or measuring the marker.

Some of the ether groups are preferred in the form of an acetal or ketal structure. Advantageouly, these groups, when hydrolysed using acids such as HCl produce the original ketone from which the ketal was formed. This is advantageous as the relatively low polarity is retained and treatment with acid does not facilitate removal of the marker, which is a big drawback of prior art markers. In other words, hydrolysis of the ketal group will not enable acid extraction of the marker but will convert it to a different form which will remain in solution in the diesel and which can be analysed.The strategy of the present invention is that the detection or evidence trail for following up on laundered diesel would start with the parent compound but would also include by-products or daughter compounds produced by the laundering process. They would be sufficiently distinctive to prove the presence of the parent marker.

With the marker of the present invention, the main portion of the marker is left behind in the diesel by treatment with acid and can still be detected.Because of the availability of a range of analytical techniques as mobile units, this opens up the range of materials which can be used as markers. For example, the markers in this patent can be easily analysed by High Pressure Liquid Chromatography (HPLC) or Gas Chromatography (GC) instruments.

### Detailed Description of the invention

If organic compounds are ranked in order of polarity, ether and ester and imodoyl groups are among the less polar compounds and closer to the polarity of alkanes of which diesel is composed. In the compounds of the invention the electronegative oxygen and nitrogen atoms are protected by attaching other groups to shield them and reduce their polarity. The imodoyl group is as shown in the following compounds:

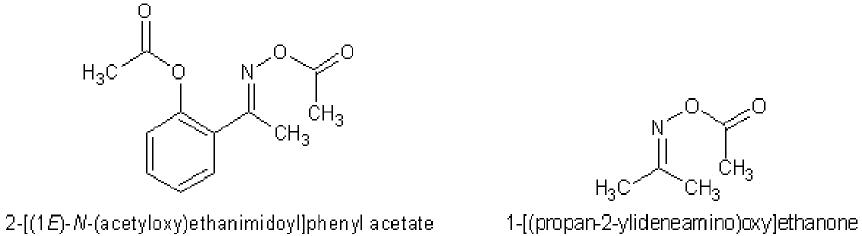

This will make it more difficult to remove the markers by absorption or by other chemical methods, which might be used in laundering. Esters and ethers and imodoyl compounds are relatively non-reactive compared to conventional fuel marker compounds, for example diazo dyes such as Solvent Red 24 or hydroxyl groups such as are found in quinizarin.

The marker compounds in this invention are based on structures which, while being of relatively low polarity are distinctly different from any compound naturally found in diesel. Such markers will take advantage of advanced methods of analysis, not based solely on colour.

For example, the markers in this invention can be easily analysed by High Pressure Liquid Chromatography (HPLC) or by Gas Chromatography (GC). Portable HPLC and GC instruments are available. (e.g. SRI Instruments Model 210D HPLC is portable and is designed for easy one-person use in the field). This is an important requirement for customs officers who must make an on the spot decision on whether or not a road vehicle is using marked diesel.

The approach of the present invention is to build markers around groups which are less polar than the commonly used groups used in markers. The following list shows some common structures in order of polarity, most polar group.

| **Group name** | **Common example** | **Structure** |
|---|---|---|
| Diazo | Azobenzene | 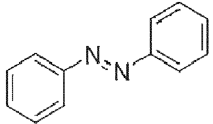 |
| Amide | Acetamide | 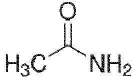 |
| Acid | Acetic acid | 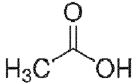 |
| Alcohol | Ethanol | CH₃CH₂OH |
| Ketone | Acetone | 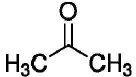 |
| Aldehyde | Acetaldehyde | 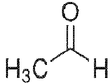 |
| Amine | Ethylamine | CH₃CH₂NH₂ |
| Imodoyl | | 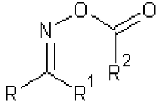 |
| Ester | Ethyl acetate | 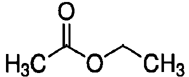 |
| Ether | Diethyl ether | 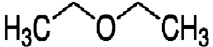 |
| Alkane | Pentane | CH₃(CH₂)₃CH₃ |

Polar organic compounds are more likely to be solids and not liquids at room temperature. They are more prone to being removed by passing through adsorbents such as silica gel or alumina. The more polar an organic compound, the lower its solubility in diesel, which is largely non-polar.

Low polar organic compounds have greater solubility in non-polar solvents such as diesel. They are more likely to be liquids and not solids at room temperatures.

Accordingly the marker structures of the present invention are based around ester or ether type groups attached to hydrocarbon groups, either alkane or benzene like structures.

The following show some general structures of the markers in this patent along with specific examples of structures.

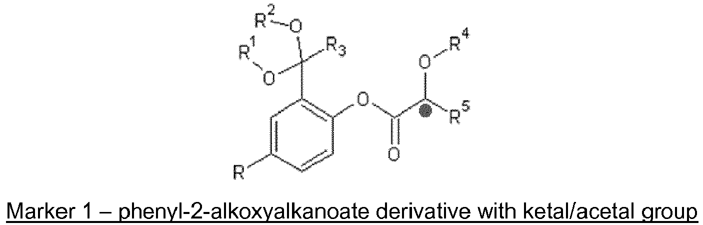

The above general structure shows a compound with ether groups, R-O-R (preferable ketal/acetal groups), an ester group, -COOR and a chiral centre marked with a red dot (depending on the groups R₁ and R₂, there may be a second chiral centre.)

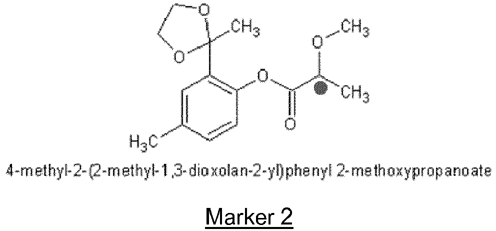

The above Marker 2 structure shows a specific structure based on the general formula Marker 1.

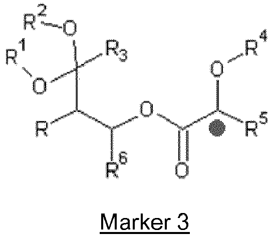

The above general structure shows a compound with ether groups, R-O-R (preferable ketal/acetal groups), an ester group, -COOR and one chiral centre marked with a red dot.

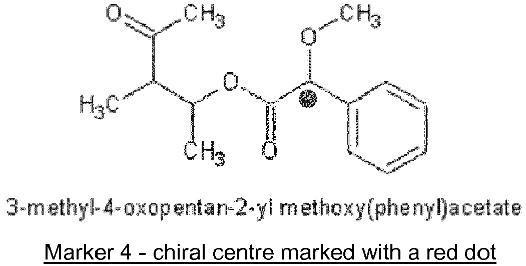

The above Marker 4 structure shows a specific structure based on the general formula Marker 2.

A range of markers can be synthesised from simple and readily available starting materials.

For example Marker 2 can be produced from lactic acid and an alkyl hydroxy acetophenone by a number of simple steps, including esterification between the acid group and the hydroxyl group on the other molecule.

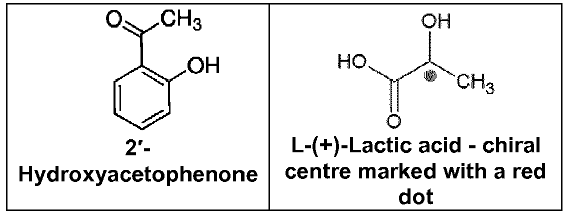

For example Marker 4 can be produced from lactic acid and 4-hydroxy-3-methylpentan-2-one by a number of simple steps including esterification between the acid group and the hydroxyl group on the other molecule.

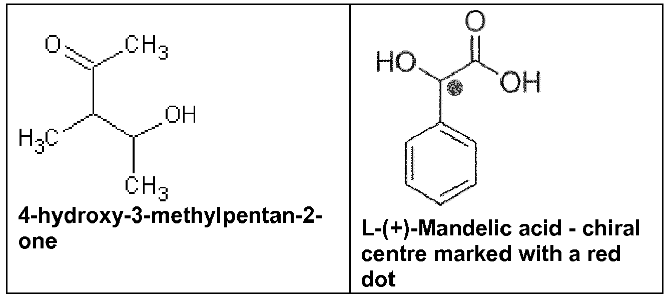

The final product can be easily varied by changes in the various starting materials or other components. A limited number of starting material types can lead to a wide permutation of final products, all of which would have the potential to mark diesel or other hydrocarbons.

The criminals who carry out laundering to remove the markers are skilled in developing and applying a range of technologies to do this. In general when the revenue authorities introduce a new marker, there is a learning curve while these criminals develop new laundering methods. The markers of the present invention will be much more difficult to remove because their properties are so close to those of fuels in polarity etc. If some extreme chemical methods are used the marker may be broken down to some of its component parts but these component parts would still remain in the fuel as an identifier. It is possible to visualise that the detection or evidence trail for following up on laundered fuel would start with the parent marker compound but would also include the by-product materials produced by the laundering operation. They would be sufficiently distinctive to prove the presence of the parent marker.

For example, treatment of Maker 2 with acid may hydrolyse the ketal group formed from the ketone. This would give Marker 2B as a by-product which would still remain in solution in the diesel.

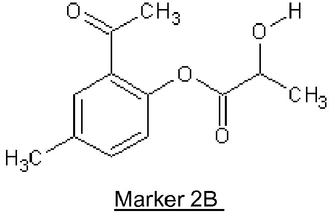

This is in contrast to Solvent Yellow 124, the Euromarker.

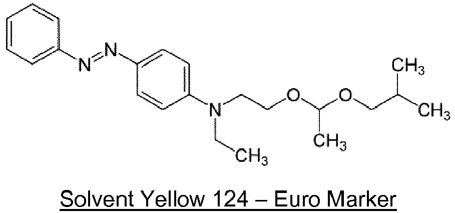

When treated with acid, it produces a water-soluble coloured form of the dye, which is extracted into acid. This is the basis of the roadside test. In doing this, it is removed from the diesel.

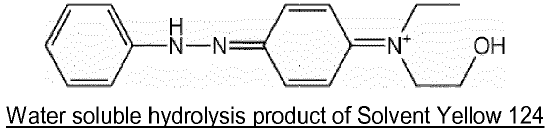

It is worth noting that the above water soluble form has a very similar structure to Butter Yellow (methyl yellow), which is toxic and a carcinogen suspect. The water soluble form is shown above in the protonated form.

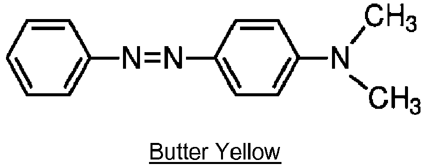

This highlights the risks associated with the use of diazo compounds compared to the compounds used as markers in this invention.

To defeat the laundering criminals it is necessary to keep a step ahead of them at all times.

The markers of the invention will facilitate this by enabling regular replacement of the original markers with other markers from within the family of compounds. This will force the laundering criminals to be continually in a process of catching up. For example, if a new marker were introduced each year as part of a budget announcement, then the criminals would also have to develop a new solution each year.

Introducing a change to the marker each year should not cause legal difficulties, as the presence of any marker, new or old, would still be evidence of an offence.

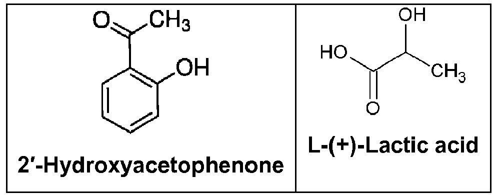

2-hydroxyacetophenone may be reacted with lactic acid to give the phenyl lactate ester. This is then methylated, optionally with dimethylsulfate, to protect the -OH group and then reacted with 1,3-propane diol, or ethylene glycol, to give the 6- or 5- sided ketal derivative which will reduce the polarity of the compound.

Preferably the final product would have an alkyl group, either linear or not, to give increased compatibility with diesel or kerosene. In this case there is an iso-propyl group attached to the benzene ring in the final product. This could be a tert-butyl or octyl group or decyl group.

But a final product without any added alkyl chain attached would also be acceptable.

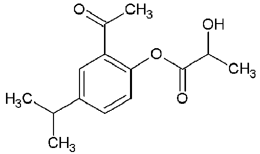

This is methylated to give: 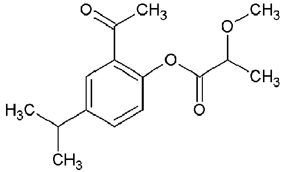 and with Ketal formation gives:-

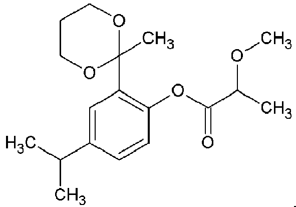

Alternatively, the following compound could be produced

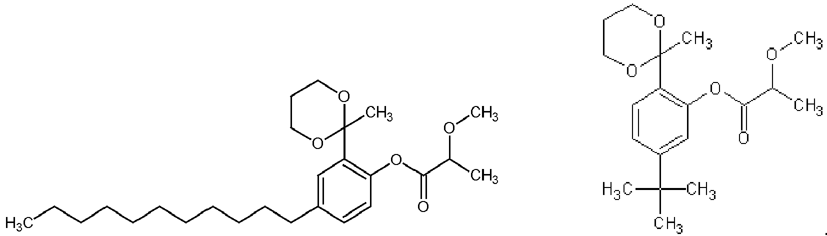

In addition the compounds of the invention have a number of desirable features which can be tested in the laboratory and which could form the basis for a roadside test: -
● Presence of optically active compound, either in pure form or in racemic mix, which can be deleted optically
● Detection by HPLC with an optically active detector, non-optically active compounds will not interfere
● The presence of Acetal or Ketal groups which under controlled conditions, can be converted to a ketone, for which roadside testing by reacting with DNPH in detector tube is possible.
● A suite of compounds based on core structures which can be easily manufactured from available starting and simple chemical operation, formation of esters etc.
● The strategy of the invention is that the detection or evidence trail for following up on laundered diesel would start with parent compound but would also include by-product or daughter produced by the laundering process. They would be sufficiently distinctive to prove the presence of the parent marker.
● Much greener chemistry compared to manufacture of diazo dyes
● More suitable for localised small scale production
● Greater flexibility to change, tweak structure on a regular basis, e.g. each year at time of annual budget to stay a few steps ahead of criminals who launder fuels.

The current alternative synthesis of the desired products is depicted in scheme 1.

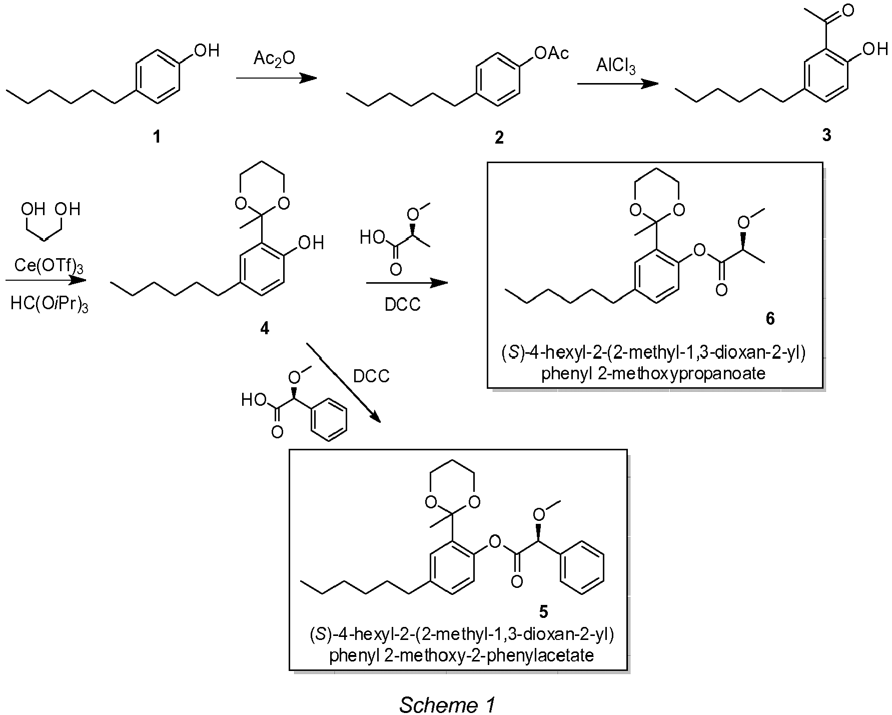

The third batch of the Fries re-arrangement gave 61.1 g of compound 3. ¹H-NMR was in accordance with structure.The acetal formation was carried out using 1,3-propanediol, tri-isopropylorthoacetate and a catalytic amount of cerium triflate in two batches. The test reaction was carried out with 4.4 g of starting material. The crude product containing 10% of starting material was purified by column chromatography. Pure compound 4 was isolated in 58% yield. The second batch in which 61 g of compound 3 was converted gave 62 g of crude compound 4 and contained 14% of compound 3, according to ¹H-NMR. This batch was purified by column chromatography.

Two test reactions on 1 g scale were carried out for the formation of the final products 5 and 6, using DCC and catalytic DMAP in CH₂Cl₂. Analysis by TLC showed that the in both cases the product was formed. The products were purified by column chromatography.

75 g of 4-hexyl phenol was acetylated to compound **2,** in quantitative yield. The Fries rearrangement to compound **3** was carried out in 3 batches. A batch of 5 g of compound 2 gave 4.4 g of compound **3** (87% yield). The second batch gave 24.9 g of compound 3 as a brown oil. ¹H-NMR was in accordance with structure.

The words "comprises/comprising" and the words "having/including" when used herein with reference to the present invention are used to specify the presence of stated features, integers, steps or components but does not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. A compound comprising the general structure (I) 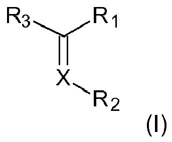
in which, X is N or O;
R³ is OR⁴ or R⁴;
wherein when X is N, R¹ is H, R⁵ or OR⁶; R² is OC(O)R⁷; and R⁴ is R⁵, OR⁵ or aryl, which is unsubstituted or substituted with at least one of R⁵ or OR⁶, OC(O)R⁷, C(O)R⁸, OR⁹, C(H)(OR')₂ or C(R¹⁰)(OR')₂;
wherein when X is O, R² is absent; R¹ is H, R⁵ or OR⁶, CH(OR⁸)R⁹; and R⁴ is R⁵, OR⁵ or aryl, which is unsubstituted or substituted with at least one of R⁵ or OR⁶, OC(O)R⁷, C(O)R⁸, OR⁹, C(H)(OR')₂ or C(R¹⁰)(OR')₂;
wherein R⁵, R⁶, R⁷, R⁸, R⁹, and R¹⁰ are independently selected from linear or branched C₁ - C₂₀ alkyl or OH; and
R' is independently selected from linear or branched C₁ -C₂₀ alkyl, or may together form a cyclic aliphatic ring having from 2 to 40 carbon atoms.

2. A compound as claimed in claim 1 wherein the compound has general structure (Ia) or (Ib) 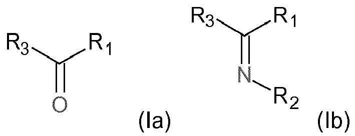 and wherein R⁵, R⁶, R⁷, R⁸, R⁹, and R¹⁰ are independently selected from linear or branched C₁ -C₁₀ alkyl; and R' is independently selected from linear or branched C₁ -C10 alkyl, or may together form a cyclic aliphatic ring having from 2 to 20 carbon atoms.

3. A compound as claimed in claim 1 wherein the compound has general structure (Ic) or (Id) or (Ie) or (If) 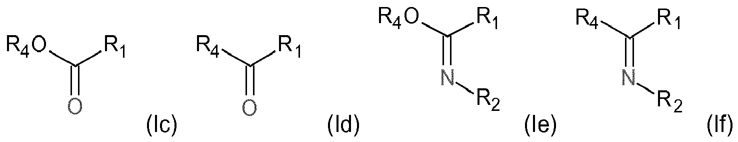 and wherein R⁵, R⁶, R⁷, R⁸, R⁹, and R¹⁰ are independently selected from linear or branched C₁ -C₆ alkyl; and R' is independently selected from linear or branched C₁ -C₆ alkyl, or may together form a cyclic aliphatic ring having from 2 to 12 carbon atoms.

4. A compound as claimed in claim 1 wherein the compound has general structure (Ig) or (Ih) or (Ii) or (Ij)

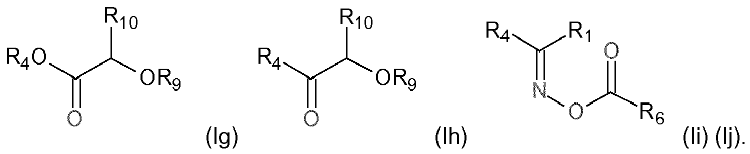

5. A compound as claimed in claim 3 or 4 wherein R⁵, R⁶, R⁷, R³, R⁹, and R¹⁰ are independently selected from linear or branched C₁ -C₃ alkyl; and R' is independently selected from linear or branched C₁ -C₃ alkyl, or may together form a cyclic aliphatic ring having from 2 to 6 carbon atoms.

6. A compound as claimed in claim 5 wherein R⁵, R⁶, R⁷, R⁸, R⁹, and R¹⁰ are independently CH₃.

7. A compound as claimed in any preceding claim wherein R4 is preferably aryl, for example phenyl.

8. A compound as claimed in claim 7 wherein the compound may be of general formula type: 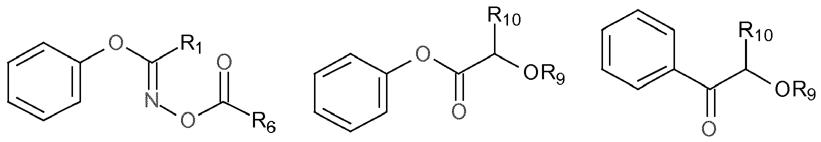 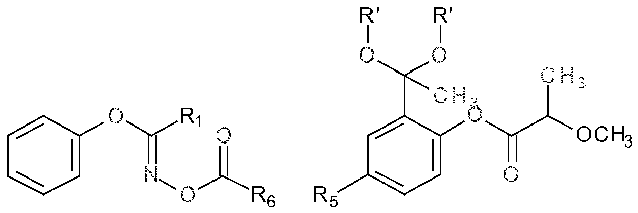 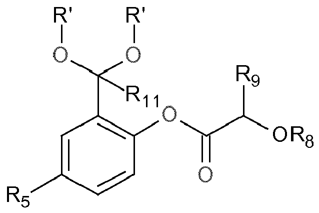 wherein the R1, R6, R9 and R10 are as described above.

9. A compound as claimed in claim 7 wherein R4 is group OR5, which is preferably a propylene chain and the compound has general formulas:-

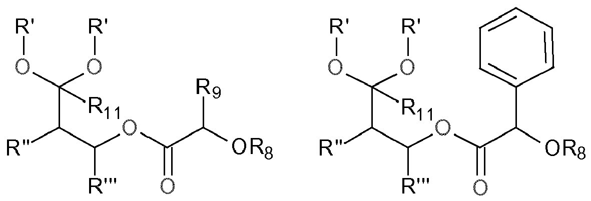

10. A compound as claimed in claim 7 wherein the compounds include one selected from the group consisting of:

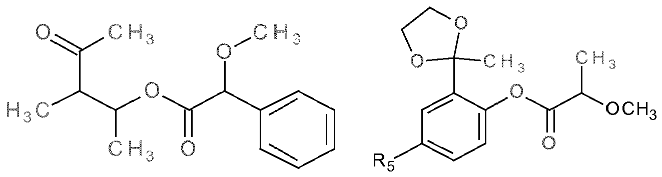

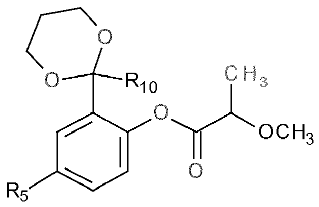

11. A compound as claimed in any preceding claim selected from the group consisting of:

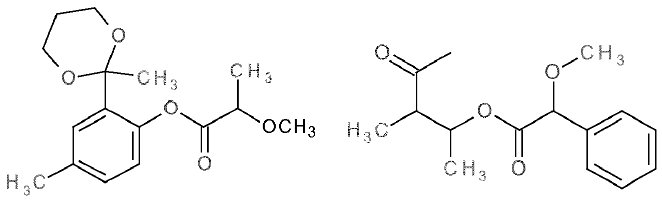

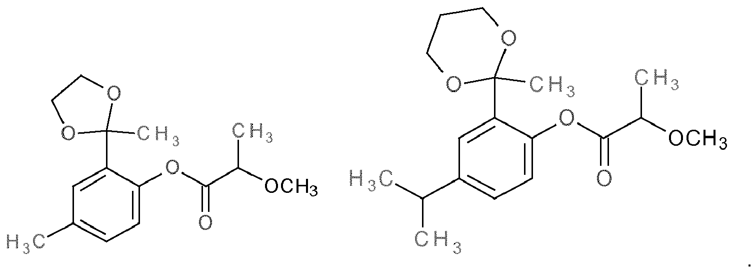

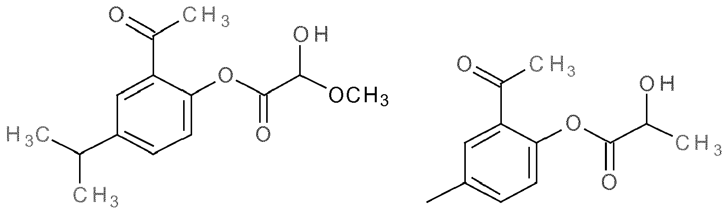

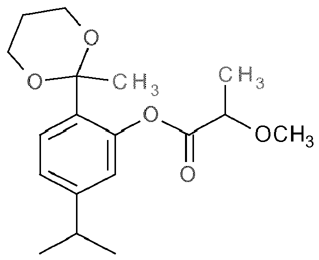

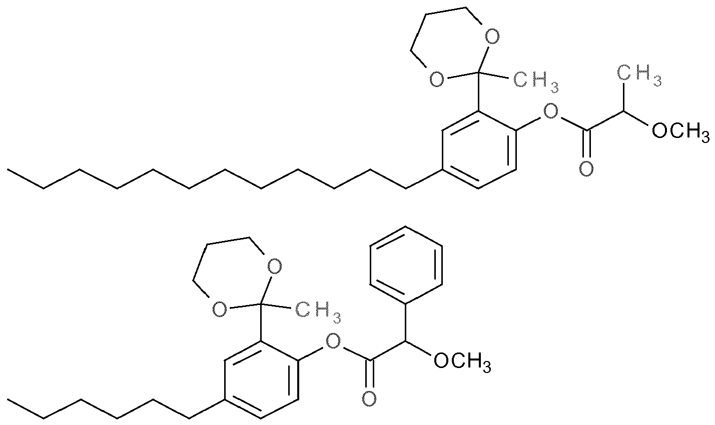

12. Use of a compound as claimed in any preceding claim as a fuel marker.

13. Use of a compound as claimed in claim 12 wherein the fuel is diesel, petrol or paraffin fuel.

14. A fuel comprising a compound as claimed in any of claims 1 to 11.

15. A method of detecting fuel laundering comprising detection of a compound as claimed in any of claims 1 to 11, or a by-product of such a compound.
